# EUROPEAN PATENT SPECIFICATION

(11) **EP 1 400 564 B1**
(45) Date of publication and mention of the grant of the patent: **02.02.2005**
(21) Application number: 02090326.6
(22) Date of filing: 11.09.2002
(51) Int. Cl.: C08L 33/20, C08F 220/44, C08F 220/58, B01D 71/40

(54) **Polymeric composition, material produced thereof and their applications**
Polymermasse, Materialien hieraus und deren Anwendungen
Compositions polymériques, matériaux obtenus à partir de celles-ci et leurs applications

(43) Date of publication of application: 24.03.2004
(73) Proprietor: GKSS Forschungszentrum, 21502 Geesthacht (DE)
(72) Inventor: Groth, Thomas, Dr., 10439 Berlin (DE); Seifert, Barbara, Dr., 10249 Berlin (DE); Albrecht, Wolfgang, Dr., 14513 Teltow (DE); Malsch, Günter, Dr., 14513 Teltow (DE); Courtney, James McNiven, Prof., Kirkintilloch Glasgow G66 4EA (GB); Gaylor, John David Stewart, Dr., Giffnock Glasgow G46 7JN (GB); Grant, Mary Helen, Dr., Bearsden Glasgow G61 3ET (GB)
(74) Representative: Schneider, Henry, Dipl.-Ing.

(56) References cited:
- EP-A- 0 559 902
- DE-A- 10 030 307
- US-A- 6 130 303
- HONIGER J ET AL: "Semiautomatic macroencapsulation of large numbers of porcine hepatocytes by coextrusion with a solution of AN69 polymer" BIOMATERIALS, ELSEVIER SCIENCE PUBLISHERS BV., BARKING, GB, vol. 21, no. 12, June 2000 (2000-06), pages 1269-1274, XP004195837 ISSN: 0142-9612

## Description

The invention relates to a novel polymeric composition, a method for its production, and its use for the production of materials to be employed in medical or biological applications, particularly for biohybrid or bioartificial organs, involving a direct contact of the material with fluids and/or cells and/or tissues.

Biohybrid or bioartificial organs are designed to support or temporarily replace human organs with malfunctions. The hybrid usually comprises membranes having "passive" separation, filter and carrier functions and also living organ cells having "active" transport and secretion functions.

In artificial organs like the artificial kidney so far only the passive separation and filter function of the organ is replaced with the help of specialised polymeric membranes. However, in the healthy kidney the filtration process is followed by an active re-adsorption process realised by the kidney epithelium. In this process, ingredients of the primary urine and water are transported against a concentration gradient from the primary urine back to the blood. Furthermore the kidney epithelium possesses an endocrine function responsible for release of erythropoietin, vitamin D hormones, angiotensins II, kinins and renins contributing to the homeostasis of the organism.

Analogue situations exist for the liver being one of the most important endocrine gland of the organism secreting hormones and other proteins (albumins, α- und β-globulins, fibrinogen, prothrombin, clotting factors) with storage function for glycogen, proteins, vitamins, beside its detoxifying function. It is clear that with the help of pore membranes only passive filtration and sorptive functions of this organ responsible for detoxification can be achieved and used for therapeutic purposes.

Therefore the immobilisation of organ cells in so-called biohybrid organs was proposed to use the active transport and secretion function of viable cells for supporting or replacing the functions of diseased organs. Until now the achieved results are of little therapeutic value because on the one hand the viability of cells can not be maintained for the necessary time period and on the other hand the functionality of the immobilised cells achieved so far does not correspond to the functionality of the natural organ.

One of the main reasons of this unsatisfactory functionality originates from the membrane in terms of being a carrier of anchorage dependent cells. Corresponding to the present knowledge the membrane carrier should possess the following properties for optimal functionality of the cells:
1. In general there are insufficient number of primary cells from the patient's own organ available. Therefore donor cells of human or animal origin have to be used in biohybrid organs. In every case a shielding of the patient from allogenic or xenogenic cells in the bioreactor is necessary to prevent activation of the host immune system. This immune isolation limits the maximum pore size of the membrane. Antibodies, complement factors, viruses etc. should not pass through the membrane. On the other hand the permeability of the membrane should permit the exchange of substances necessary for supply to the cells (nutrients, some proteins, such as albumin, gases, toxins to be removed) and removal of the products of the cells (cell metabolites, specific proteins, etc.).
2. Organ cells like hepatocytes are adhesion-dependent and require an extracellular matrix at the contact sites to the membrane where they are anchored. Only in this way they maintain viable over a longer time period, develop their normal morphology and function and organise in polar, tissue-like structures. Hence, the membrane must allow the moderate adsorption of extracellular matrix proteins without major conformational changes and allow their subsequent structural organisation. Consequently, the membrane surface contacting the tissue cells has to fulfil extreme requirements to be tissue compatible.

Depending on the organ to be supported or replaced and the requirements stated in paragraph 1 and 2 the protein adsorption capacity of the membrane has a crucial importance. Endocrine organs produce proteins that have to be delivered into the blood stream without major adsorption during passage through the membranes to fulfil their function in the human body. Thus, the membrane may have only a moderate adsorption capacity for proteins.

DE 100 30 307 A discloses a material consisting of 70 to 95 mole-% of acrylonitrile monomers and N-vinyl pyrrolidone as comonomer. Membranes produced of this material by a phase inversion process are intended to be used in medical or biological applications.

Further, a hydrogel is known, which is produced by a phase inversion process of a polymer solution containing a copolymer of acrylonitrile and sodium methallylsulfonate in a suitable solvent (J. Honiger et al.: Biomaterials 21, 2000, 1269-74). The material is used for macroencapsulation of allogenic and xenogenic hepatocytes.

EP 0 559 902 A describes a membrane produced of a copolymer which is constituted of 90 to 97 mole-% of acrylonitrile monomer and at least one comonomer unit such as vinyl acetate or 2-N,N-diethylaminoethyl methacrylate. The membranes are used for the filtration/separation of organic substances.

It is an object of the present invention to provide a composition and a material made of the composition, both suitable for being used in applications involving direct contact with cells, especially tissue cells. The material made from the composition shall support moderate adsorption of the cells and support their natural functions. Moreover, the composition should be suitable for preparing membranes with pore sizes that enable exchange of substances, which are necessary for the supply of the cells. At the same time, the pore size should ensure an efficient immune isolation to shield a patient from the allogeneic or xenogeneic cells attached to the membrane.

This and other objects are accomplished by a polymeric composition comprising at least one acrylic copolymer constituted of 90 to 99 mole-% of acrylic (AC) monomer units and of at least one anionic water-soluble comonomer unit, which is 2-acrylamido-2-methyl-propane sulfonic acid (AMPS).

Furthermore this and other objects are accomplished by a polymeric composition comprising at least one polyacrylonitrile copolymer constituted of 90 to 99 mole-% of acrylonitrile (AN) monomer units and of at least one water-soluble comonomer unit.

Molar contents of AMPS in the copolymer of 1 to 10 mole-%, particularly of 3 to 5 mole-%, have been shown to be favourable with respect to the desired product characteristics as outlined above.

The polymeric composition according to the present invention shows high biocompatibility for many cells, including tissue cells, particularly epithelial cells. Therefore, the composition is suitable for the production of biomaterials, such as membranes, films or coatings, which can be used in medical or biological applications involving direct contact of the material with body fluids and/or cells and/or tissues, particularly with hepatocytes. Cells that are immobilised at the material can easily be fed by a fluid stream, which supplies the cells with nutrients thereby enabling exchange of substances into the cells and outside the cells.

It has been shown that materials, produced of the composition can advantageously be used in biohybrid or bioartificial organs, where they work as selectively permeable separating membrane and carrier for adhesion-dependent tissue cells, such as liver, pancreas, lung cells. As shown by experiments on liver cells immobilised on membranes according to the invention, these cells maintain a high degree of natural cytochrome dependent testosterone and ethoxyresorufin metabolism and also express glutathione-S-transferase.

Due to their poor cytotoxicity and low affinity for protein adsorption, materials made up of the polymeric composition can also be used for medical applications within the body. For example they may be applied for implants or biosensors.

The composition can easily be produced by radical copolymerisation of AN and the comonomer in presence of a suitable radical initiator, such as ammonium peroxodisulfate, all dissolved in an aprotic solvent, such as dimethylformamide. To obtain the appropriate product characteristics the reaction is preferably carried out at a total concentration of monomers in the reaction solution in the range of 1 to 5 mole/l and a concentration of the radical initiator of about 0,4 to 4 mmole/l.

Membranes, preferably having a rate of water flux through the membrane in the range of 1 to 10 I/m²hkPa, particularly of about 2 I/m²hkPa, and a cut-off in the range of 150 to 1,000 kDa, particularly of 200 to 600 kDa, even more particularly of 300 to 400 kDa are favourably obtained by a phase-inversion process from the copolymers according to the invention. In detail, a casting solution, which contains a polymeric composition at a solid content of for example 15 to 25 weight-% dissolved in a suitable solvent, is prepared. Then the solution is casted on a support or extruded through a suitable nozzle, particularly of the so-called tube-in-orifice type; and the cast or the extruded solution is coagulated by a wet or wet-dry-wet process in a coagulation bath to form an asymmetric membrane. Preferably, the membrane is subsequently subjected to a wet post-treatment in water or steam.

Other preferred embodiments of the invention are disclosed in the dependent claims.

The invention is described in more detail by way of examples and the following figures:
- Figure 1: Cytotoxicity test of membranes prepared according to examples 3 and 4,
- Figure 2: MTT test of 3T3 fibroblast growth on membranes according to examples 3 and 4 (control - MC).

### Example 1: Polymer synthesis

The copolymers according to the present invention generally comprise poly-acrylonitrile (PAN) derivatives functionalised by the anionic comonomer 2-acrylamido-2-methyl-propane sulfonic acid (AMPS).

The introduction of the functionality was achieved by means of free-radical random copolymerisation techniques to yield copolymers having well adjusted composition ratios and good membrane forming properties. Homopolymerised PAN was included in the studies as reference material.

Before use, AN basic monomer (MERCK, >99 % purity) was inert fractional distilled. AMPS comonomer (ALDRICH, 98 % pur.) was applied without further purification. Dimethylformamide (DMF, ALDRICH, 99,8 % pur.) was inert vacuum distilled at 40 mbar/b.p.60°C before application as aprotic solvent in polymer synthesis. Free radical initiators 2,2'-azoisobutyronitrile (AIBN, recrystallised twice from water at 30°C) or ammonium peroxidisulfate (APS, fractional crystallised from ethanol at 40°C) were used in homo- and copolymerisations, respectively.

Solution polymerisation initiated by free radicals was carried out as an upscaling procedure (5 l batch size) using a conventional five-necked double-walled jacket glass reactor with an outer thermostatting. From the recipe ingredients, first the main portion of aprotic DMF solvent was filled into the reactor and heated up to the desired temperature under continuous stirring (150 r.p.m.) in an inert atmosphere. Subsequently, AN and the comonomer and finally the initiator, dissolved in the remaining DMF, were added into the batch. The reaction time was individually regulated from 360 min (PAN) to 1800 min (AMPS) to obtain the required conversions. In the case of APS initiated copolymer series, the polymerisation temperature was adjusted to 45°C, whereas in the case of AIBN initiated AN homopolymerisation the temperature was 54°C as found by kinetic calculations for adjusting the homo- and copolymer molecular weights.

Because of initiator differentiation it can be assumed definitely that the PAN reference obtained with AIBN is free of any functionalities, even in initiator terminal position.

The applied total monomer concentration of [M] = 3,8 mole/l for PAN and 5,0 mole/l for AMPS copolymer includes well graduated molar ratios of the comonomer in the feed, ranging from zero for PAN reference up to 5,0 mole-% for the AMPS enriched feed.

The total monomer concentrations and initiator concentrations of 8 mmole/l (AIBN for PAN) or 4 mmole/l (APS for copolymer series) were balanced in order to obtain number average molecular weights higher than 30,000 g/mole for obtaining membrane forming polymers suitable for the required ultrafiltration pore structure.

After the end of reaction, polymer solutions were poured into a large excess of ethanol used as precipitant. The precipitated polymer was filtered, manifold washed and rinsed with dehydrated ethanol, which was predried on air before exhaustive vacuum drying, until constant weight was reached.

### Example 2: Polymer characteristics

The polymers synthesised from AN and AMPS according to example 1 were characterised for copolymer composition by elementary analysis and number average molecular weight by membrane osmometry. PAN was used as reference material. The results of this analysis are shown in Table 1.

**Table 1:**

| Polymer characteristics. x_{AMPS}, _{Pol.} = comonomer concentration in copolymer from N-elementary analysis, M_{n,Osm.} = molecular weight of copolymer measured in 0,1 n NaNO₃/DMF-electrolyte solution. | | | |
|---|---|---|---|
| **Polymerisation pattern** | **Abbreviation** | **Polymer characteristics** | |
| | | **X**_{**AMPS, Pol.**} **[mole %]** | **M**_{**n,Osm.**} **[g/mole x10**^{**-3**}**]** |
| **PAN** | **PAN** | - | 48.5 |
| **P(AN/AMPS)1/1** | **AMPS1/1** | 2.9 | 33.5 |
| **P(AN/AMPS)1/2** | **AMPS1/2** | 3.2 | 32.3 |
| **P(AN/AMPS)1/3** | **AMPS1/3** | 2.9 | 45.3 |
| **P(AN/AMPS)2/1** | **AMPS2/1** | 5.2 | 57.3 |

### Example 3: Membrane formation

Membranes were formed by a phase inversion process from a solution of the polymers according to examples 1 and 2 in dimethylformamide. The polymer concentration varied from 10 to 22,5 wt-%. The solution was cast on a non-woven support (AMPS1/1, 1/2 and 2/1) or directly on a stainless steel band with a casting slit of 300 µm and a drawing speed of 2 to 4 m/min. After passage through a water precipitation bath at 5 °C or room temperature membranes were tempered in water for 10 min (Table 2).

**Table 2:**

| Membrane formation data. | | | | |
|---|---|---|---|---|
| **Membrane** | **Polymer concentration in solution [wt-%]** | **Temperature of polymer solution** | **Precipitation bath** | **Tempering** |
| **PAN** | 15 | 1-2 °C | 5 °C | 10 min 90 °C |
| **AMPS1/1** | 22.5 | RT | RT | 10 min 65 °C |
| **AMPS1/2** | 22.5 | RT | RT | 10 min 65 °C |
| **AMPS2/1** | 22.5 | RT | RT | 10 min 65 °C |

The formation of membranes from AMPS1/3 with polymer concentration less than 15% for getting a larger pore structure was not manageable because the material is highly swellable. Hence blend membranes from PAN and AMPS1/3 were formed with varying pore structures. It was assumed that PAN builds a connected polymer network surrounded by the copolymer AMPS1/3. Data on this membrane formation process are given in Table 3.

**Table 3:**

| Blend membrane formation data. | | | | |
|---|---|---|---|---|
| **Membrane** | **Polymer concentration in solution [wt %]** | **Temperature of polymer solution** | **Precipitation bath** | **Tempering** |
| **PAN/AMPS1/3 (1:1) 288** | 15 | RT | RT | 10 min 50 °C |
| **PAN/AMPS1/3 (1:1) 289** | 10 | RT | RT | 10 min 50 °C |
| **PAN/AMPS1/3 (3:1) 290** | 10 | RT | RT | 10 min 50 °C |

### Example 4: Membrane sieving characteristics

### 4.1 Water permeability and contact angles

The membranes formed according to example 3 had water permeabilities and water contact angles as given in Table 4. Water fluxes of the membranes were determined in an ultrafiltration device - a Berghoff stirred cell - with distilled water. Water fluxes of AMPS membranes were less than those of PAN membranes mainly due to the higher polymer concentration for AMPS membrane formation resulting in a more dense structure. Compared to PAN, for the AMPS copolymers significant differences in the advancing (wetting) and receding (dewetting) water contact angles were measured indicating more hydrophilic surfaces of the latter materials.

**Table 4:**

| Water permeabilities and water contact angles of membranes. | | | |
|---|---|---|---|
| **Membrane** | **Water flux** | **Water contact angle** | |
| | **[l/m**^{**2**}**hkPa]** | **advancing[°]** | **receding[°]** |
| **PAN** | 4.02 | 59.5 | 43.0 |
| **AMPS1/1** | 0.022 | 47.3 | 13.8 |
| **AMPS1/2** | 0.020 | 54.4 | 17.1 |
| **AMPS2/1** | 0.020 | 30.5 | 15.6 |
| **PAN/AMPS1/3 (1:1) 288** | 0.200 | 0 | 0 |
| **PAN/AMPS1/3 (1:1) 289** | 0.838 | 0 | 0 |
| **PAN/AMPS1/3 (3:1) 290** | 2.205 | 0 | 0 |

The water fluxes of AMPS blend membranes increase with lower polymer concentration and higher PAN content in the casting solution. The separation parameters of PAN/AMPS1/3 290 are close to the requirements of a membrane for nutrition supply and waste disposal for tissue cells (water flux about 2 l/m²hkPa) and can be adjusted towards desired values.

For the active surfaces of the blend membranes advancing and receding water contact angles of 0° were found indicating highly hydrophilic surfaces.

### 4.2 Membrane performance

To demonstrate the ability to change selectively porosity and transport properties during membrane formation process, AMPS blend membranes with different polymer concentrations and compositions in casting solutions were formed according to examples 1 to 3.

Sieving properties of membranes were determined in a Berghoff stirred cell with distilled water and a solution of a test substance mixture (dextrans with molecular weights from 650 to 215.000). Molecular size distributions of dextrans in permeates, in retentates and in the original solution were determined using gel permeation chromatography (GPC).

From the results of GPC the average pore diameter (D₅₀), the maximal pore diameter (D₁₀₀) and the corresponding molecular weights (cut-off) were calculated by assuming a logarithmic normal distribution of the pore size (Table 5).

**Table 5:**

| Separation ability and performance characteristics of PAN/AMPS copolymer blend membranes. | | | | |
|---|---|---|---|---|
| **Membrane** | **Pore characteristics** | | | |
| | **D**_{**50**} **[nm]** | **D**_{**100**} **[nm]** | **σ** | **Cut-off [kDa]** |
| **PAN/AMPS1/3 (1:1) 288** | 3.73 | 7.84 | 0.2573 | 23.2 |
| **PAN/AMPS1/3 (1:1) 289** | 8.73 | 20.08 | 0.2889 | 188 |
| **PAN/AMPS1/3 (3:1) 290** | 20.75 | 33.56 | 0.1668 | 588 |

The separation parameters of PAN/AMPS1/3 (3:1) are close to the requirements of a membrane for immunoisolation (cut-off 300 to 400 kDa) and can be changed towards desired values.

. Membrane permeability was investigated by measurements of albumin permeability. A 3 mM albumin solution was used as test solution and PBS as buffer on the sink side of a membrane test cell. Samples were taken every 20 min from the sink side and were measured for an increase in albumin concentration by a Lowry assay (Table 6).

**Table 6:**

| Permeability for albumin of PAN/AMPS copolymer blend membranes. | |
|---|---|
| **Membrane** | **Membrane permeability k**_{**o**} **[cm/min]** |
| **PAN/AMPS1/3 (1:1) 288** | 3.3 x 10⁻⁵ ± 2.8 x 10⁻⁵ |
| **PAN/AMPS1/3 (1:1) 289** | 10.5 x 10⁻⁵ ± 7.9 x 10⁻⁵ |
| **PAN/AMPS1/3 (3:1) 290** | 10.9 x 10⁻⁵± 10.4 x 10⁻⁵ |

### Example 5: General biocompatibility - contact with 3T3 fibroblasts

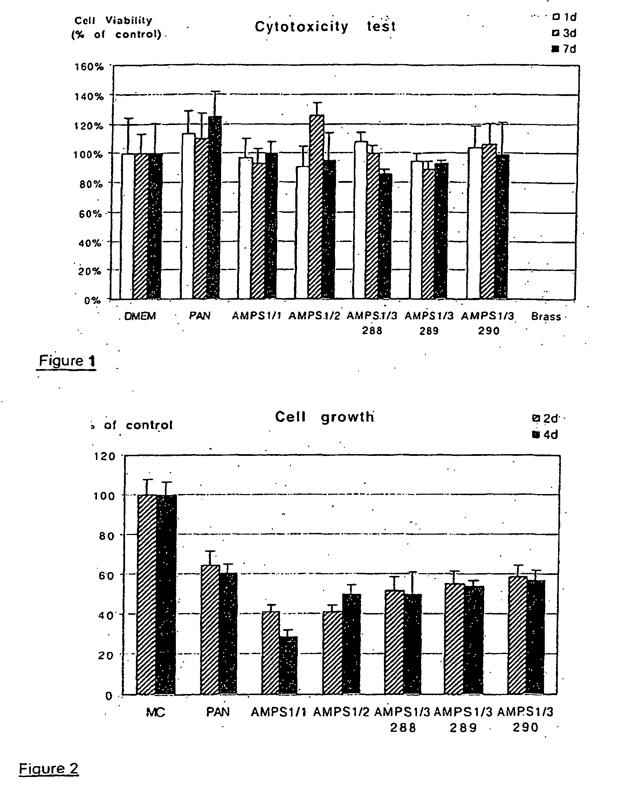

For testing the general biocompatibility of membranes produced according to examples 1 to 4, experiments with 3T3 fibroblast were performed. The cytotoxicity was tested according to ISO10993-5 with extracts of membranes obtained after 1, 3 and 7 days of incubation of samples in cell culture medium (DMEM). As shown by neutral red test, confluent layers of 3T3 fibroblasts did not show inhibition of viability after 24 hours incubation with these extracts indicating no cytotoxic properties (Figure 1).

Membranes prepared according to examples 1 to 4 were tested for biocompatibility after 2 and 4 days of direct contact to 3T3 fibroblasts. Results were compared to a reference material (Millicell® cell culture insert by Millipore). Figure 2 shows growth rates of 3T3 fibroblasts estimated by the MTT assay. Compared to the reference material PAN, copolymer membranes have less favourable surfaces properties for fibroblasts. The first batch (AMPS1/1) yields still significantly lower growth values for these cells.

### Example 6: Membranes in contact with hepatocytes

### 6.1. Plating efficiency and maintenance of cytochrome content

Primary cultures of hepatocytes are renowned for their phenotypic instability. Moreover, the concentration of total cytochrome P450 and the activities of its isoenzymes are recognised as the most labile functions, extremely sensitive to the culture environment. Thus, hepatocytes were used to assess the maintenance of functions of the primary cultures on membranes according to examples 1 to 4. If the membranes permitted maintenance of this function it is assumed that they were likely to be suitable for supporting most of hepatocyte functions.

Hepatocytes were isolated from male Sprague-Dawley rats (180-220 g) by collagenase perfusion. Viability of 80-90 % of isolated cells was confirmed by Trypan Blue exclusion. Hepatocytes were seeded at 1.6 x 10⁶ viable cells per membrane (membrane discs 40 mm in diameter) or 3 x 10⁶ viable cells per collagen-coated (25 µg/cm² Type I collagen isolated from rat tail tendons) 60 mm diameter polystyrene Petri dishes (for control dishes). Cells were cultured in Chee's medium supplemented with 5 % (v/v) foetal calf serum, in an atmosphere of 5 % CO₂ in air. Four hours after seeding, the cultures were washed to remove unattached cells, and fresh medium was supplied at this time and once more after 24 h. Samples were taken for assessment of the membranes after 48 h. Plating efficiency was determined by measuring total cell protein by the Lowry assay. Cytochrome P450 and b5 were measured by a modified method of Omura and Sato. Results are given in Table 7.

**Table 7:**

| Plating efficiency and cytochrome maintenance of hepatocytes cultured for 48 h on the membranes (control = collagen-coated polystyrene Petri dishes, control cell values = 133.42 pmole/mg protein for cytochrome P450 and 155.42 pmole/mg protein for cytochrome b5). Values are means ± SEM, number of experiments given parentheses. | | | |
|---|---|---|---|
| **Membrane** | **Plating efficiency** | **Cytochrome maintenance [% of control]** | |
| | **[%]** | **Cyt P450** | **Cyt b5** |
| **control** | 66.4 ±16.0 (6) | 100 | 100 |
| **PAN** | 63.2 ±12.0 (5) | 92.7 ± 43.2 (6) | 109.2 ± 38.1 (6) |
| **AMPS1/1** | 66.4 ± 16.0 (5) | 95.3 ± 16.1 (5) | 107.3 ± 22.8 (5) |

### 6.2 Cytochrome P450 dependent testosterone metabolism and O-deethylation of ethoxyresorufin (EROD)

The biocompatibility of membranes according to examples 1 to 4 in terms of supporting primary cultures of rat hepatocytes was assessed by measuring the cytochrome P450 dependent metabolism of testosterone and ethoxyresorufin.

Primary rat hepatocytes isolated according to example 6.1 were seeded with a density of 1.6 x 10⁶ cells per membrane (cultures on membrane discs, 40 mm in diameter) and 3 x 10⁶ cells per collagen-coated 60 mm diameter Petri dish as controls. Cells were cultured in Chee's medium with 5 % foetal calf serum. After 48 h in culture, cells on the membranes and controls were incubated with 100 pM testosterone in serum-free medium for 60 min. Products were analysed by HPLC. In a second test, the cells cultured for 48 h were washed, scraped into sodium phosphate buffer and homogenised . using 7 strokes of a motor driven homogeniser. 0.5 ml of the homogenates were incubated with 6 µM ethoxyresorufin in the presence of NAPDH for 10 minutes. Formation of resorufin was measured fluorimetrically.

Table 8 demonstrates a high testosterone metabolism found for rat hepatocytes on the AMPS1/1 membrane, although this functionality of liver cells on the membranes according to the invention is less than that on the collagen control. However, the range of hydroxylated metabolites formed on the AMPS1/1 membrane was the best of all membranes tested. Cells on this membrane formed 6-beta-hydroxy-, 16-alpha-hydroxy, 16-beta-hydroxytestosterone and androstenedione, whereas most of the other membranes including the PAN membrane supported only the conversion of testosterone to androstenedione. Table 9 shows that the EROD activity is higher in cells cultured on PAN or AMPS1/1 membranes than on controls.

**Table 8:**

| Total testosterone metabolites formed by cell cultures (n=3) on PAN and AMPS1/1 membranes. Controls = cells cultured on collagen-coated polystyrene Petri dishes. | |
|---|---|
| **Membrane** | **Total testosterone metabolism [nmole/h/mg protein]** |
| **Control** | 5.4 ± 0.2 |
| **PAN** | 1.5 ± 1.5 |
| **AMPS1/1** | 3.4 ± 1.5 |

**Table 9:**

| The activity of EROD in cells cultured on PAN and AMPS1/1 membranes (n=3). Controls = cells cultured on collagen-coated polystyrene Petri dishes. | |
|---|---|
| **Membrane** | **EROD activity [pmole resorufin formed/min/mg protein]** |
| **Control** | 0.6 ± 0.1 |
| **PAN** | 2.0 ± 0.2 |
| **AMPS1/1** | 2.2 ± 0.2 |

### 6.3 Glutathione-S-transferase (GST)

The expression of alpha- and pi-isoenzymes of glutathione-S-transferase (GST) changes with de-differentiation and age of liver cells in culture. With culturing time, alpha-GST expression declines whereas that of pi-GST increases. Expression of both of these GST proteins was detected in cells growing on the membranes according to the present invention by immunoblotting using specific antibodies. Table 10 shows expression of alpha-GST and pi-GST in cells grown on PAN and AMPS1/1 membranes. Alpha-GST was well maintained in cells grown on both membranes. Cells on the AMPS1/1 membrane had particularly low levels of pi-GST suggesting a higher degree of differentiation.

**Table 10:**

| Expression of alpha-GST and pi-GST in cells cultured on PAN and AMPS1/1 membranes (n=3). Controls are cells cultured on collagen-coated Petri dishes. | | |
|---|---|---|
| **Membranes** | **Alpha-GST (OD units)** | **Pi-GST (OD units)** |
| **Control** | 15.9 ± 0.6 | 1.5 ± 0.8 |
| **PAN** | 33.7±10.4 | 2.5 ± 1.9 |
| **AMPS1/1** | 24.3 ± 10.0 | 0.7 ± 0.6 |

### 6.4 Long-term performance

At day 7 and 16 of culturing hepatocytes on the AMPS1/1 membrane, cells functions were assessed by the EROD assay, by testosterone metabolism and by GST expression. The effect of collagen coating of the AMPS1/1 membrane on the function of the cells was also investigated. As for the Petri dishes control, collagen was coated at 25 µg/cm² on the membrane. The results are shown in Tables 11 and 12 for testosterone metabolism, in Table 13 for the EROD activity and in Table 14 for the GSTs, respectively. Total metabolism of testosterone was less in cells cultured on the membrane than in cells cultured on the Petri dishes at both 7 and 16 days. Collagen coating of the membrane improved the range of metabolites formed by the cells cultured for 7 days on the membrane, but made little difference after 16 days. At day 7, the collagen-coated membrane also supported cells which showed lower EROD activities, but again this effect could not be observed after 16 days. Collagen coating also improved the expression of alpha GST in cells cultured on AMPS1/1 membrane at 7 days, but not after 16 days of culture. However, there was also a high level of expression of pi GST in cells after 7 days culture on the collagen-coated membrane, but no pi-GST was detectable after 16 days with collagen coating.

Long term culture of cells on AMPS1/1 membrane showed that this membrane could maintain viable cells metabolising testosterone and ethoxyresorufin and expressing a high level of alpha GST and a low level of pi GST isoenzymes for 16 days. Collagen coating of the membrane improved its performance for up to 7 days, but by 16 days collagen coating made little difference to the enzyme activities of the cells cultured on AMPS1/1 membrane.

**Table 11:**

| Total testosterone metabolites formed by cells cultured for 7 and 16 days on the AMPS1/1 membrane, with and without collagen coating (n=3). Controls are cells on collagen-coated Petri dishes. | |
|---|---|
| **Membrane / culture time** | **Total testosterone metabolised [nmole/h/mg protein]** |
| **Control, 7 d** | 7.2 ± 0.8 |
| **AMPS1/1, 7 d** | 1.8 ± 1.0 |
| **AMPS1/1 collagen, 7 d** | 3.7 ± 0.8 |
| **Control, 16 d** | 4.4 ± 0.8 |
| **AMPS1/1, 16 d** | 2.1 ± 0.4 |
| **AMPS1/1 collagen, 16 d** | 1.6 ± 0.9 |

**Table 12:**

| Formation of metabolites from testosterone by cells cultured for 7 and 16 days on the AMPS1/1 membrane with and without collagen coating (n=3). Controls are cells on collagen-coated Petri dishes. Values are means ± SEM, the formation of each metabolite is expressed as a percentage of the total metabolites formed (see Table 11). | | | | | |
|---|---|---|---|---|---|
| **Membrane / culture time** | **6-alpha Hydroxy** | **6-beta Hydroxy** | **16-alpha Hydroxy** | **2-alpha Hydroxy** | **Androstendi one** |
| **Control, 7 d** | 9.3 ± 1.0 | 29.8±3.1 | 0 | 9.5 ± 1.0 | 51.3±5.0 |
| **AMPS1/1, 7 d** | 4.6 ± 4.6 | 30.1 ± 15.4 | 0 | 0 | 32.0 ± 16.0 |
| **AMPS1/1 collagen, 7 d** | 7.5 ± 4.0 | 30.8 ±2.6 | 0 | 3.5 ± 3.5 | 58.2 ±6.3 |
| **Control, 16d** | 3.7 ± 3.7 | 26.7 ±1.5 | 0 | 6.2 ± 3.1 | 63.4 ±4.9 |
| **AMPS1/1, 16d** | 0 | 6.8 ± 6.8 | 0 | 5.1 ± 5.1 | 88.3 ± 6.0 |
| **AMPS1/1 collagen, 16 d** | 0 | 11.8 ± 11.8 | 3.5 ± 3.5 | 0 | 51.3 ±28.9 |

**Table 13:**

| EROD activity of cells cultured for 7 and 16 days on the AMPS1/1 membrane with and without collagen coating (n = 3). Controls are cells cultured on collagen-coated Petri dishes. Values are means ± SEM. | |
|---|---|
| **Membrane / culture time** | **EROD activity [pmole resorufin formed/min/mg protein]** |
| **Control, 7 d** | 0.8 ± 0.2 |
| **AMPS1/1, 7 d** | 0.8 ± 0.7 |
| **AMPS1/1 collagen, 7 d** | 0.4 ± 0.3 |
| **Control, 16 d** | 0.3 ± 0.1 |
| **AMPS1/1, 16 d** | 0.2 ± 0.2 |
| **AMPS1/1 collagen, 16 d** | 1.0, 0.9 (n=2) |

**Table 14:**

| Expression of alpha-GST and pi-GST in cells cultured on AMPS1/1 membrane with and without collagen coating for 7 and 16 days (n=3). Controls are cells cultured on collagen-coated Petri dishes. Values are means ± SEM. | | |
|---|---|---|
| **Membrane / culture time** | **GST-alpha [OD units]** | **GST-pi [OD units]** |
| **Control, 7 d** | 11.2 ± 0.5 | 0 |
| **AMPS1/1, 7 d** | 11.9 ± 11.1 | 1 ±1 |
| **AMPS1/1 collagen, 7 d** | 24.0 ± 9.7 | 11.3 ± 2.6 |
| **Control, 16 d** | 9.7 ± 1.3 | 4.2 ± 3.7 |
| **AMPS1/1, 16 d** | 20.2 ±3.4 | 1.6 ±0.1 |
| **AMPS1/1 collagen, 16 d** | 11.6 ± 5.6 | 0 |

### 6.5 Morphology

Hepatocytes isolated and cultured according to example 6.1 were seeded on AMPS1/1 membranes according to examples 1 to 4 and incubated up to 20 days. Some of the membranes were coated with collagen before seeding the cells. As control, hepatocytes were also cultured on collagen-coated Petri dishes. The morphology of the hepatocytes on the AMPS1/1 membranes with and without collagen coating was compared with the control. Cell viability was assessed at day 7, 16 and 20 of culture using carboxyfluorescein diacetate (CFDA) and confocal microscopy. Viable cells were maintained at all time points. The membrane according to the present invention supported cell viability as well as the collagen-coated Petri dishes did. On AMPS1/1 membrane cells showed good morphology, with highly developed bile cannaliculi.

## Claims

1. A polymeric composition comprising at least one acrylic copolymer constituted of 90 to 99 mole-% of acrylic (AC) monomer units and of at least one anionic water-soluble comonomer unit, **characterized in that** the at least one comonomer is 2-acrylamido-2-methyl-propane sulfonic acid (AMPS).

2. A polymeric composition according to claim 1, comprising an acrylonitrile copolymer constituted of 90-99 mole-% of acrylonitrile (AN) monomer units and of 2-acrylamido-2-methyl-propane sulfonic acid (AMPS).

3. Composition according to claim 1 or 2, comprising AMPS with a molar content in the copolymer of 1 to 10 mole-%, particularly of 3 to 5 mole-%.

4. Method for producing a composition according to claims 1 to 3, comprising copolymerising acrylonitrile (AN) monomers with at least one anionic water-soluble comonomer in presence of a suitable radical initiator in a suitable solvent, wherein that the at least one comonomer is 2-acrylamido-2-methyl-propane sulfonic acid (AMPS).

5. Method according to claim 4, wherein the radical initiator is ammonium peroxodisulfate (APS).

6. Method according to claim 4 or 5, wherein a total concentration of monomers in the reaction solution of 1 to 5 mole/l, particularly of about 4 mole/l, and a concentration of the radical initiator of 0,4 to 4 mmole/l, particularly of about 4 mmole/l, is adjusted.

7. Method according to any one of the preceding claims 4 to 6, wherein the solvent is an aprotic solvent, particularly dimethylformamide (DMS).

8. Material produced from a composition according to claims 1 to 3 for medical or biological applications involving a direct contact of the material with fluids and/or cells and/or tissues.

9. Material according to claim 8, wherein the material is a membrane, a film or a surface coating.

10. Material according to claim 8 or 9, wherein the material is employed as support for cells, particularly tissue cells, the cells being in contact with a fluid stream, which supplies the cells with nutrients and enables exchange of substances into the cells and outside the cells.

11. Material according to claim 10, wherein the tissue cells are hepatocytes.

12. Material according to any one of preceding claims 8 to 11, wherein the material is used in biohybrid or bioartificial organs.

13. Material according to claim 12, wherein the material is a membrane with immobilised organ cells, such as liver, pancreas, lung cells, and wherein the cells are separated from the fluid stream.

14. Material according to any of the preceding claims 8 to 11, wherein the material is used for medical applications within the body, such as implants or biosensors.

15. Membrane, film or coating essentially made up of a composition according to any one of claims 1 to 3.

16. Membrane, film or coating comprising a blend of a composition according to any one of claims 1 to 3 and polyacrylonitrile (PAN).

17. Membrane according to claim 15 or 16 being formed by a phase-inversion process.

18. Membrane according to any one of preceding claims 15 to 17, wherein the membrane is an asymmetric membrane, comprising an outer dense layer having an average pore size of 1 to 50 nm, particularly of 3 to 20 nm, more particularly of 5 to 12 nm.

19. Membrane according to any one of preceding claims 15 to 17, comprising a flat or hollow fibre membrane having at least a two-layer cross sectional structure substantially consisting of a dense surface layer and a porous bulk layer having finger-like pores communicating with the dense layer.

20. Membrane according to any one of preceding claims 15 to 19, having a rate of water flux through the membrane in the range of 1 to 10 l/m²hkPa, particularly of about 2 I/m²hkPa.

21. Membrane according to any one of preceding claims 15 to 20, having a cut-off in the range of 150 to 1,000 kDa, particularly of 200 to 600 kDa, more particularly of 300 to 400 kDa.

22. Method for producing a membrane according to any one of preceding claims 15 to 21, comprising the steps of
(a) preparing a casting solution, which contains a polymeric composition according to claims 1 to 3 dissolved in a suitable solvent;
(b) casting the solution on a support or extruding the solution through a suitable nozzle; and
(c) coagulating the cast or the extruded solution in a coagulation bath to form an asymmetric membrane.

23. Method according to claim 22, wherein the asymmetric membrane is subjected to a wet post-treatment in water or steam.

24. Method according to claim 22 or 23, wherein the casting solution is prepared to have a solid content of the polymeric composition of 15 to 25 weight-%.

25. Method according to any one of preceding claims 22 to 24, wherein the solvent is a polar solvent.

26. Method according to any one of preceding claims 22 to 25, wherein the coagulation is performed by a wet or wet-dry-wet process.

27. Method according to any one of preceding claims 22 to 26, wherein the casting solution is extruded through a tube-in-orifice type nozzle.

## Patentansprüche

1. Polymere Zusammensetzung, umfassend mindestens ein acrylisches Copolymer, das aus 90 bis 99 Mol-% einer acrylischen Monomereinheit (AC) und aus mindestens einer anionischen, wasserlöslichen Comonomereinheit aufgebaut ist, **dadurch gekennzeichnet, dass** das mindestens eine Comonomer 2-Acrylamido-2-methylpropansulfonsäure (AMPS) ist.

2. Polymere Zusammensetzung nach Anspruch 1, umfassend ein Acrylnitril-Copolymer, das aus 90 bis 99 Mol-% Acrylnitril-Monomereinheiten (AN) und aus 2-Acrylamido-2-methylpropansulfonsäure (AMPS) aufgebaut ist.

3. Zusammensetzung nach Anspruch 1 oder 2, umfassend AMPS mit einem molaren Gehalt in dem Copolymer von 1 bis 10 Mol-%, insbesondere von 3 bis 5 Mol-%.

4. Verfahren zur Herstellung einer Zusammensetzung nach den Ansprüchen 1 bis 3, umfassend Copolymerisation von Acrylnitril-Monomeren (AN) mit mindestens einem anionischen, wasserlöslichen Comonomer in Gegenwart eines geeigneten Radikalstarters in einem geeigneten Lösungsmittel, wobei das mindestens eine Comonomer 2-Acrylamido-2-methylpropansulfonsäure (AMPS) ist.

5. Verfahren nach Anspruch 4, wobei der Radikalstarter Ammoniumperoxodisulfat (APS) ist.

6. Verfahren nach Anspruch 4 oder 5, wobei eine Gesamtkonzentration von Monomeren in der Reaktionslösung von 1 bis 5 mol/l, insbesondere von etwa 4 mol/l, und eine Konzentration des Radikalstarters von 0,4 bis 4 mmol/l, insbesondere von etwa 4 mmol/l, eingestellt wird.

7. Verfahren nach einem der vorhergehenden Ansprüche 4 bis 6, wobei das Lösungsmittel ein aprotisches Lösungsmittel, insbesondere Dimethylformamid (DMS) ist.

8. Material, hergestellt aus einer Zusammensetzung nach den Ansprüchen 1 bis 3, für medizinische oder biologische Anwendungen, die einen direkten Kontakt des Materials mit Fluiden und/oder Zellen und/oder Geweben einschließen.

9. Material nach Anspruch 8, wobei das Material eine Membran, eine Folie oder eine Oberflächenbeschichtung ist.

10. Material nach Anspruch 8 oder 9, wobei das Material als Träger für Zellen, insbesondere Gewebezellen, verwendet wird und die Zellen in Kontakt mit einem Fluidstrom stehen, welcher die Zellen mit Nährstoffen versorgt und einen Austausch von Substanzen in und aus den Zellen ermöglicht.

11. Material nach Anspruch 10, wobei die Gewebezellen Hepatozyten sind.

12. Material nach einem der vorhergehenden Ansprüche 8 bis 11, wobei das Material in biohybriden oder bioartifiziellen Organen verwendet wird.

13. Material nach Anspruch 12, wobei das Material eine Membran mit immobilisierten Organzellen ist, wie etwa Leber-, Bauchspeicheldrüsen-, Lungenzellen, und worin die Zellen von dem Fluidstrom separiert sind.

14. Material nach einem der vorhergehenden Ansprüche 8 bis 11, wobei das Material für medizinische Anwendungen innerhalb des Körpers eingesetzt wird, wie etwa für Implantate oder Biosensoren.

15. Membran, Folie oder Beschichtung, im Wesentlichen bestehend aus einer Zusammensetzung nach einem der Ansprüche 1 bis 3.

16. Membran, Folie oder Beschichtung, umfassend einen Blend aus einer Zusammensetzung nach einem der Ansprüche 1 bis 3 und Polyacrylnitril (PAN).

17. Membran nach Anspruch 15 oder 16, hergestellt durch ein Phaseninversionsverfahren.

18. Membran nach einem der vorhergehenden Ansprüche 15 bis 17, wobei die Membran eine asymmetrische Membran ist, die eine äußere dichte Schicht mit einer mittleren Porengröße von 1 bis 50 nm umfasst, insbesondere von 3 bis 20 nm, speziell von 5 bis 12 nm.

19. Membran nach einem der vorhergehenden Ansprüche 15 bis 17, umfassend eine Flach- oder eine Hohlfasermembran mit mindestens einer zweischichtigen Querschnittsstruktur, die im Wesentlichen aus einer dichten Oberflächenschicht und einer porösen Hauptschicht besteht, weiche fingerartige, mit der dichten Schicht kommunizierende Poren aufweist.

20. Membran nach einem der vorhergehenden Ansprüche 15 bis 19 mit einer Wasserflussrate durch die Membran im Bereich von 1 bis 10 l/m² hkPa, insbesondere etwa 2 l/m²hkPa.

21. Membran nach einem der vorhergehenden Ansprüche 15 bis 20 mit einem Cut-off im Bereich von 150 bis 1.000 kDa, insbesondere von 200 to 600 kDa, speziell von 300 bis 400 kDa.

22. Verfahren zur Herstellung einer Membran nach einem der vorhergehenden Ansprüche 15 bis 21, umfassend die Schritte
(a) Herstellen einer Gießlösung, die eine in einem geeigneten Lösungsmittel gelöste polymere Zusammensetzung nach den Ansprüchen 1 bis 3 enthält;
(b) Gießen der Lösung auf einen Träger oder Extrudieren der Lösung durch eine geeignete Düse und
(c) Koagulieren des Gusses oder der extrudierten Lösung in einem Koagulationsbad unter Ausbildung einer asymmetrischen Membran.

23. Verfahren nach Anspruch 22, wobei die asymmetrische Membran einer nassen Nachbehandlung in Wasser oder Dampf unterzogen wird.

24. Verfahren nach Anspruch 22 oder 23, wobei die Gießlösung mit einem Feststoffgehalt der polymeren Zusammensetzung von 15 bis 25 Gew.-% hergestellt wird.

25. Verfahren nach einem der vorhergehenden Ansprüche 22 bis 24, wobei das Lösungsmittel ein polares Lösungsmittel ist.

26. Verfahren nach einem der vorhergehenden Ansprüche 22 bis 25, wobei die Koagulation durch ein Nass- oder ein Nass-Trocken-Nass-Verfahren ausgeführt wird.

27. Verfahren nach einem der vorhergehenden Ansprüche 22 bis 26, wobei die Gießlösung durch eine Düse eines Rohr-in-Mündung-Typs (tube-in-orifice type) extrudiert wird.

## Revendications

1. Composition polymère, comprenant au moins un copolymère acrylique, qui est constitué de 90 à 99 mol-% d'un motif monomère acrylique (AC) et d'au moins un motif comonomère anionique hydrosoluble, **caractérisée en ce que** le comonomère dont au moins un est présent est l'acide 2-acrylamido-2-méthylpropanesulfonique (AMPS).

2. Composition polymère suivant la revendication 1, comprenant un copolymère d'acrylonitrile qui est constitué de 90 à 99 mol-% de motifs monomères d'acrylonitrile (AN) et d'acide 2-acrylamido-2-méthylpropanesulfonique (AMPS).

3. Composition suivant la revendication 1 ou 2, comprenant de l'AMPS en proportion molaire dans le copolymère de 1 à 10 mol-%, en particulier de 3 à 5 mol-%.

4. Procédé de production d'une composition suivant les revendications 1 à 3, comprenant la copolymérisation de monomères d'acrylonitrile (AN) avec au moins un comonomère anionique hydrosoluble en présence d'un initiateur radicalaire convenable dans un solvant convenable, le comonomère dont au moins un est présent étant l'acide 2-acrylamido-2-méthylpropanesulfonique (AMPS).

5. Procédé suivant la revendication 4, dans lequel l'initiateur radicalaire est le peroxodisulfate d'ammonium (APS).

6. Procédé suivant la revendication 4 ou 5, dans lequel sont ajustées une concentration totale de monomères dans la solution réactionnelle de 1 à 5 mol/1, en particulier d'environ 4 mol/1, et une concentration de l'initiateur radicalaire de 0,4 à 4 mmol/1, en particulier d'environ 4 mmol/l.

7. Procédé suivant l'une des revendications 4 à 6 précédentes, dans lequel le solvant est un solvant aprotique, en particulier le diméthylformamide (DMS).

8. Matériau, produit à partir d'une composition suivant les revendications 1 à 3, destiné à des applications médicales ou biologiques qui impliquent un contact direct du matériau avec des fluides et/ou des cellules et/ou des tissus.

9. Matériau suivant la revendication 8, ce matériau étant une membrane, une feuille ou un revêtement de surface.

10. Matériau suivant la revendication 8 ou 9, ce matériau étant utilisé comme support pour des cellules, en particulier des cellules de tissus et les cellules sont en contact avec un courant de fluide qui alimente ces cellules en substances nutritives et qui permet un échange de substances vers les cellules et à partir de cellules.

11. Matériau suivant la revendication 10, dans lequel les cellules de tissus sont des hépatocytes.

12. Matériau suivant l'une des revendications 8 à 11 précédentes, ce matériau étant utilisé dans des organes biohybrides ou bioartificiels.

13. Matériau suivant la revendication 12, ce matériau étant une membrane à cellules d'organes immobilisées telles que, par exemple, des cellules de foie, de pancréas, de poumon et où les cellules sont séparées du courant de fluide.

14. Matériau suivant l'une des revendications 8 à 11 précédentes, ce matériau étant utilisé pour des applications médicales à l'intérieur du corps, par exemple pour des implants ou des biocapteurs.

15. Membrane, feuille ou revêtement, constitué principalement d'une composition suivant l'une des revendications 1 à 3.

16. Membrane, feuille ou revêtement, comprenant un mélange d'une composition suivant l'une des revendications 1 à 3 et d'un polyacrylonitrile (PAN).

17. Membrane suivant la revendication 15 ou 16, produite par un procédé d'inversion de phase.

18. Membrane suivant l'une des revendications 15 à 17 précédentes, cette membrane étant une membrane asymétrique qui comprend une couche externe dense dont les pores ont un diamètre moyen de 1 à 50 nm, en particulier de 3 à 20 nm, notamment de 5 à 12 nm.

19. Membrane suivant l'une des revendications 15 à 17 précédentes, constituée d'une membrane plane ou d'une membrane à fibres creuses ayant au moins une structure transversale à deux couches, qui est principalement constituée d'une couche superficielle dense et d'une couche principale poreuse qui présente des pores digités communiquant avec la couche dense.

20. Membrane suivant l'une des revendications 15 à 19 précédentes, l'eau traversant cette membrane à un débit de 1 à 10 l /m²hkPa, notamment d'environ 2 l/m²hkPa.

21. Membrane suivant l'une des revendications 15 à 20 précédentes, ayant un cut-off dans la plage de 150 à 1000 kDa, en particulier de 200 à 600kDa , notamment de 300 à 400 kDa.

22. Procédé de production d'une membrane suivant l'une des revendications 15 à 21 précédentes, qui comprend les étapes consistant
(a) à préparer une solution à couler, qui contient une composition polymère suivant les revendications 1 à 3 dissoute dans un solvant convenable ;
(b) à couler la solution sur un support ou à extruder la solution à travers une filière convenable et
(c) à faire coaguler la solution coulée ou la solution extrudée dans un bain de coagulation en réalisant une membrane asymétrique.

23. Procédé suivant la revendication 22, dans lequel la membrane asymétrique est soumise à un post-traitement par voie humide dans l'eau ou dans la vapeur d'eau.

24. Procédé suivant la revendication 22 ou 23, dans lequel la solution à couler est produite avec une teneur en matière solide de la composition polymère de 15 à 25 % en poids.

25. Procédé suivant l'une des revendications 22 à 24 précédentes, dans lequel le solvant est un solvant polaire.

26. Procédé suivant l'une des revendications 22 à 25 précédentes, dans lequel la coagulation est conduite par un procédé par voie humide ou par un procédé par voie humide/sèche/humide.

27. Procédé suivant l'une des revendications 22 à 26 précédentes, dans lequel la solution à couler est extrudée à travers une filière du type à tube-dans-orifice (tube-in-orifice type).
